# EUROPEAN PATENT APPLICATION

(11) **EP 1 371 321 A1**
(43) Date of publication of application: **17.12.2003**
(21) Application number: 03013079.3
(22) Date of filing: 10.06.2003
(51) Int. Cl.: A61B 1/00, A61B 1/04, A61B 1/05

(54) **Electronic endoscope for stereoscopic endoscopy**

(30) Priority: 14.06.2002 JP 2002174163
(71) Applicant: Fuji Photo Optical Co., Ltd., Saitama-shi, Saitama (JP); Ai Systems, Ltd., Saitama-shi, Saitama (JP)
(72) Inventor: Ando, Kunio, c/o Ai Systems, Ltd., Saitama-shi, Saitama (JP)
(74) Representative: Urner, Peter, Dipl.-Phys.

(57) **Abstract**

An electronic endoscope (30; 40) includes an illumination guide (37U, 37D) and a pair of right and left image pickup devices (34R, 34L) each comprising an objective lens system (31LR, 31LL) and a solid state image sensor (33R, 33L) that are all incorporated in an elongated cylindrical sheath (35) and operates to produce right and left optical images of an object which are displayed on a monitor as a three-dimensional image and is provided with a mounting fixture (36) which has a pair of mounting bores (36H) formed side by side therein for holding the right and left solid state image pickup devices (34R, 34L) in the mounting bores (36H), respectively, so as thereby to align the right and left solid state image pickup devices (34R, 34L) side by side in a diametrical direction of the sheath (35) and is shaped so as to provide arched spaces (35U, 35D) between the mounting fixture (36) and the sheath (35) for receiving the illumination guide (37U, 37D) therein when it is fitted in the sheath (35).

## Description

The present invention relates to an electronic endoscope for a stereoscopic endoscope system and, more particularly, to an electronic endoscope suitably used as a laparoscope in a stereoscopic endoscope system that essentially includes an elongated rigid tubular sheath having a reduced outer diameter for insertion into a human or animal body to view therein or to perform surgical procedures.

In recent years, endoscopic surgical operations on or endoscopic direct visual examinations of abdominal organs within a human or animal body are widely performed using laparoscopes. Such an endoscopic surgical operation or an endoscopic direct visual examination is performed by introducing a laparoscope and, if necessary, forceps into an abdominal cavity through a relatively small incision in the skin.

In the endoscopic surgical operation or the endoscopic direct visual examination, in light of the fact that stereoscopic visual images of the interior of a human body are more accurate and realistic than conventional two-dimensional images, many stereoscopic endoscope systems including laparoscopes that provide stereoscopic vision of the internal of the body have been proposed.

Such a stereoscopic endoscope system typically includes an electronic endoscope such as an electronic laparoscope which has a elongated rigid tubular sheath provided with a pair of objective lens systems positioned at a distal end thereof and a pair of relay lens systems as image guides for transmitting right and left images of the interior of a patient body formed by the objective lens systems to a pair of video cameras set up separately from the electronic endoscope. The images of the interior of the patient body are displayed as a three-dimensional image on a monitor through the video camera. This type of electronic endoscope is unfavorable because the viewer is provided with poor visual perception.

In the meanwhile, with recent advance in miniaturization of solid state charge coupled devices (CCDs) as an imaging device, electronic endoscopes that include a pair of image pickup means each of which comprises an objective lens system and a CCD image sensor disposed, basically in place of the conventional relay lens systems and video camera, in the distal end of the elongated rigid tubular sheath have been put into practical use and prevails in the field of endoscopic surgical operations and endoscopic direct visual examinations. Such an electronic endoscope for a stereoscopic endoscope system produces high quality images (e.g., on a monitor connected to the system) which a properly-equipped user/viewer of the system will perceive as three -dimensional images having stereoscopic depth. With this system, the viewer is provided with visual perception because of stereopsis.

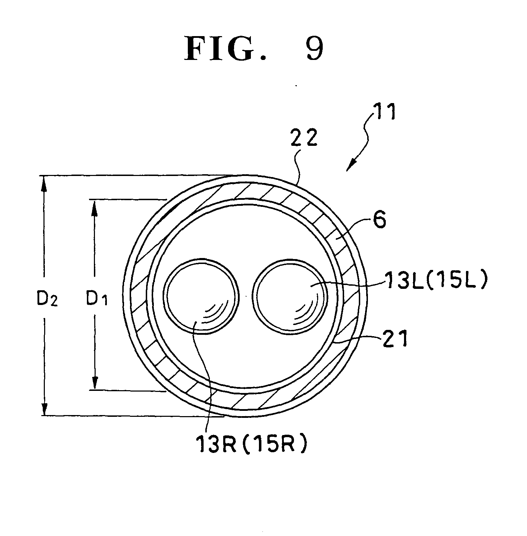

Referring is made to Figures 8 and 9 for the purpose of providing a brief background that enhances an understanding of a stereoscopic endoscope system including an electronic endoscopic instrument. Referring to Figure 8 showing a stereoscopic endoscope system exemplified as a conventional system, the stereoscopic endoscope system comprises an electronic laparoscope 1 as an endoscopic instrument, a light source unit 2, a video signal processing unit 3 and a video display or monitor 4. The electronic laparoscope 1 generally includes an elongated rigid cylindrical sheath 1A, that has a double-barreled structure such as described in detail later, for insertion into an interior of a body cavity of a patient, i.e. a human body or animal body, to view therein or to perform surgical procedures, a pair of or right and left image pickup devices 15R and 15L disposed in close proximity to an distal end 11 of the sheath 1A for generating and transmitting images of the cavity from within the body and an illumination or light guide 6 comprising a generally cylindrically tubular-shaped bundle of optical fibers and extending through the sheath 1A. The electronic laparoscope 1 at a proximal end further includes a handling unit 1B provided with a light guide connector 7 for optically connecting the light guide 6 to the light source unit 2 and a relay connector 9 for electrically connecting the image pickup devices 15R and 15L to the video signal processing unit 3 through respective signal lines 8.

The right and left image pickup devices 15R and 15L, that correspond to right and left eyes of the viewer respectively, are located within the sheath 1A in close proximity to the distal end 11 opposite to the proximal end where the handling unit 1B is attached. Each image pickup device 15L or 15R comprises an objective lens system 13R or 13L which gathers a right or a left optical image of an object, i.e. the cavity of the patient body, illuminated with light from the light guide 6 and a CCD image sensor 14L or 14R as a solid state image sensor which converts the optical image incident thereupon into video signals. The right and left video signals are transmitted to and processed by the signal processing unit 3 in an ordinary manner. The processed right and left video signals are distributed to a remote monitoring unit 4 to display right and left images which can be perceived as a three-dimensional image having stereoscopic depth when observed through a viewing device (not shown) such as a specially-designed eyeglasses which are known in various forms and may take any form well known in the art.

Referring to Figure 9 showing an exemplary configuration of the distal end 11 of the laparoscope shown in Figure 8, the sheath 1A of the electronic laparoscope comprises internal and external rigid cylindrical tubular barrels 21 and 22 which have outer diameters D1 and D2, respectively, different sufficiently to provide a cylindrical tubular space therebetween for receiving the light guide 6 in the form of a tubular-shaped bundle of fibers therein. The right and left image pickup devices 15R and 15L are fixedly mounted in the internal cylindrical barrel 21 in close proximity to the distal end 11. In this example, the interior of an abdominal cavity of the patient body is illuminated with light diverging in the form of an annular ring from the light guide 6.

Although the electronic laparoscope shown in Figures 8 and 9 is desirable for physicians and surgeons in the viewpoint of homogeneous or uniformly distributed illumination over the interior of an abdominal cavity of a patient body with light, nevertheless, it is distasteful to patients to make a relatively large incision in the skin for introducing the electronic laparoscope, stereoscopic or non-stereoscopic, whose sheath 1A has to unavoidably have a large outer diameter (the outer diameter D2 of the external cylindrical barrel 22) due to the configuration of the electronic laparoscope in which the cylindrically tubular-shaped light guide 6 surrounding the inner cylindrical barrel 21 is covered with the outer cylindrical barrel 22 as seen in Figure 9.

It is therefore an object of the present invention to provide an electronic endoscope for stereoscopic endoscope systems which includes an elongated cylindrical sheath for insertion into the interior of a body cavity or a hollow organ of a human or animal body made significantly thin.

It is another object of the present invention to provide an electronic endoscope for stereoscopic endoscope systems which produces images on a monitor connected to the system perceived as three-dimensional images having stereoscopic depth by a properly-equipped viewer of the system even though an elongated cylindrical sheath of the endoscope for insertion into the interior of a body cavity or a hollow organ of a human or animal body is made as thin as possible.

The above objects of the present invention are achieved by an electronic endoscope, suitably used as a laparoscope, for stereoscopic endoscope systems that includes illumination guide means such as a optical fiber bundle optically connected to a light source for illuminating an interior of a hollow body object and a pair of right and left image pickup means, each comprising an objective lens system and a solid state image sensor such as a chip of charge coupled device (CCD), for gathering right and left optical images of the hollow body object, respectively, and converting the right and left optical images into video signals to display the left and right images on a monitor connected to the system which a properly-equipped viewer of the system perceives as a three-dimensional image having a stereoscopic effect. The electronic endoscope comprises an elongated cylindrical rigid sheath for insertion into the interior of the hollow body object and a mounting fixture that is fitted in the elongated cylindrical rigid sheath in proximity to an distal end of the elongated cylindrical sheath and has a pair of mounting bores arranged side by side to hold the right and left solid state image pickup means side by side therein in a diametrical direction of the elongated cylindrical sheath. The mounting fixture is cut off partially at either one side, more preferably opposite sides, of an axial plane including both center axes of the mounting bores so as to define an arched space or spaces between the mounting fixture and the elongated cylindrical sheath on one or both sides of the mounting fixture for receiving the illumination guide means therethrough. In this instance, the light guide means comprising a number of optical fibers is bundled so as to have an exit end in conformity with the arched space.

The electronic endoscope is preferably provided with an anterior optical element fixedly installed in the elongated cylindrical sheath at the distal end commonly through which the right and left image pickup means gather right and left optical images of the hollow body object. The anterior optical element comprises a converging lens forming a common part of the objective lens systems of the left and light image pickup means and is configured in conformity with the front of the mounting fixture.

The electronic endoscope may further be provided a transparent protective plate configured in conformity with the arched space and installed in the elongated cylindrical sheath at the distal end for protecting a distal end of the illumination guide means. The transparent protective plate or a pair of the transparent protective plates are complementary in shape to the single converging lens to form a substantially completely circular anterior optical element capable of being snugly fitted into the elongated cylindrical sheath at the distal end. The single converging lens and the transparent protective plate or plates are cemented to each other to form an integral anterior optical element.

The electronic endoscope may further be provided with light blocking means disposed at an interface between the converging lens and the illumination guide means or between converging lens and the transparent protective plate or plates for preventing stray light emanating from the illumination light guide from entering the converging lens. The light blocking means may comprise one of a light blocking film disposed at the interface, a black coating applied to the cut-off sides of the conversing lens, and a black adhesive for cementing the converging lens and the illumination guide means or the transparent protective plates together.

The circular anterior optical element, that comprises the single converging lens forming a common part of both right and left objective lens systems and the transparent protective plate or plates, may be borne down by a peripheral edge of the elongated cylindrical sheath clinched radially inwardly at the distal end. Alternatively, the elongated cylindrical sheath may be provided with an annular flange extending radially inwardly from the distal end thereof. In this instance, the mounting fixture with the right and left image pickup means attached thereto is installed into the elongated cylindrical sheath only from the proximal end.

With the electronic endoscope of the present invention, as the mounting fixture has a shape of substantial circle cut off at least partially at either one side, more preferably opposite sides, there is provided an arched space or spaces between the mounting fixture and the elongated cylindrical sheath at either one or both sides of the mounting fixture in which the illumination guide means is received. This configuration effectively improves the availability of space of the interior of the elongated cylindrical sheath at the distal end and, consequently, the elongated cylindrical sheath of the electronic endoscope is allowed to be significantly thin.

The circular anterior optical element comprising the single converging lens and the transparent protective plate or plates are borne down by a clinched peripheral edge of the elongated cylindrical sheath clinched at the distal end or an annular flange formed at the distal end of the elongated cylindrical sheath. Alternatively, the circular anterior optical element may be stopped an annular flange extending radially inwardly from the distal end of the elongated cylindrical sheath. The circular anterior optical element prevents an adhesive with which a great number of fibers forming the light guide are bonded together from deteriorating on contact with chemical solutions such as cleaning and disinfectant solutions whenever the electronic endoscope is washed in disinfectant after use. This is because, adhesive deterioration often leads to optical and/or mechanical local damages and/or deterioration of air-tightness of the distal ends of the light guides which cause failure of image quality and a problem of reliability of the electronic endoscope depending on the degree of adhesive deterioration.

The light blocking means disposed at an interface between the converging lens and the illumination guide means or between the converging lens and the transparent protective plate or plates prevents stray light emanating from the illumination light guide from entering the converging lens even in the case where the light guide is located in close proximity to either one or both of the right and left image pickup means in the elongated cylindrical sheath made thin. This results in high quality three-dimensional images.

The above and other objects and features of the present invention will be clearly understood from the following detailed description when read with reference to the accompanying drawings, wherein the same numerical symbols have been used to denote same or similar parts or mechanisms throughout the drawings, and in which:
Figure 1 is a perspective view, partly broken away, showing a configuration of a portion of an electronic endoscope in proximity to the distal end thereof for a stereoscopic endoscope system according to an embodiment of the present invention;
Figure 2 is an end view of the electronic endoscope seen from the distal end in which an anterior optical lens element is removed;
Figure 3 is a longitudinal cross-sectional view of the electronic endoscope according to the embodiment in a plane along line III-III of Figure 1 that includes a center line of an elongated cylindrical sheath of the electronic endoscope and is perpendicular to an axial plane including optical axes of a pair of image objective systems of the electronic endoscope;
Figure 4 is a perspective view, similar to Figure 1, showing a configuration of an electronic endoscope according to an alternative embodiment of the present invention;
Figure 5 is a perspective exploded view of an anterior optical element used in the electronic endoscope of the alternative embodiment;
Figure 6 is a longitudinal cross-sectional view, similar to Figure 3, of the electronic endoscope according to the alternative embodiment in a plane taken along line VI-VI of Figure 4 that includes a center line of an elongated cylindrical sheath of the electronic endoscope and is perpendicular to an axial plane including optical axes of a pair of image objective systems of the electronic endoscope;
   and
Figure 7 is a cross-sectional view, similar to Figure 6, showing a variant of the electronic endoscope according to the alternative embodiment.

In the following description, parts or units which are not of direct importance to the invention and parts or units which are purely of conventional construction will not be described in detail. For example, details of the light source unit, the video signal processing unit, the video display or monitor unit, etc., which necessary to the stereoscopic endoscope system will not set out in detail since their construction and operation can easily be arrived at by those skilled in the art.

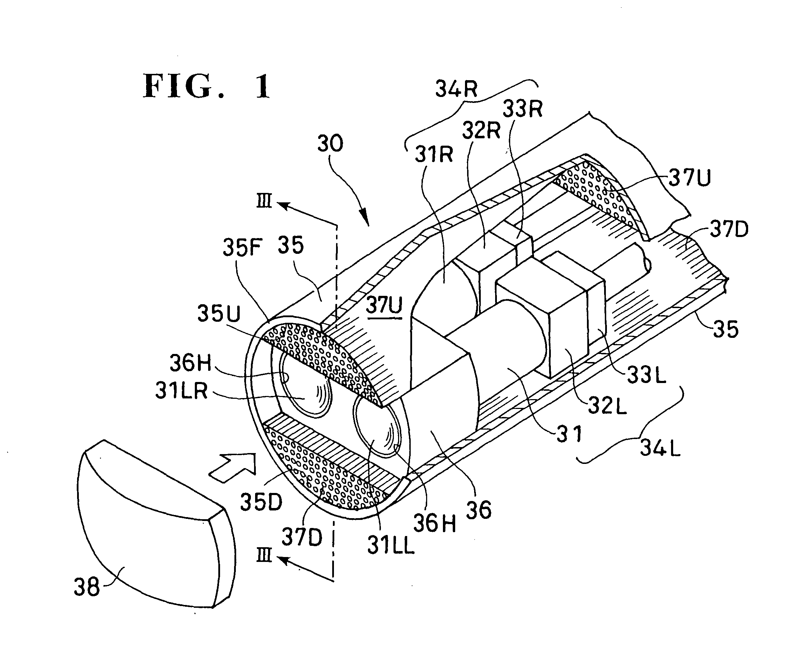

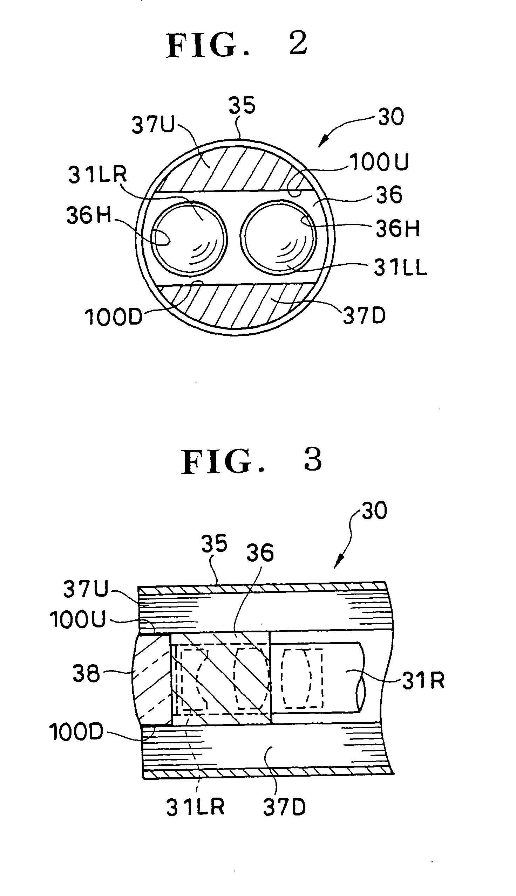

Referring now to the drawings in detail and, in particular, Figures 1 through 3 showing a front portion of an electronic endoscope 30 used typically as a laparoscope for a stereoscopic endoscope system according to a preferred embodiment of the present invention, the electronic endoscope 30 basically comprises an elongated hollow cylindrical sheath 35 (which is hereafter referred to as a cylindrical sheath for simplicity) made of a rigid tubular barrel for insertion into the interior of an abdominal cavity of a human or animal body, a pair of image pickup devices 34R and 34L operative as right and left eyes of a viewer, respectively, (which are referred to as right and left image pickup devices) for gathering right and left images of the abdominal cavity, and a pair of light guides 37D and 37U for transmitting light generated by a light source and illuminating the interior of the abdominal cavity. The left image pickup device 34L comprises an objective lens system 31LL built in a lens barrel 31L operative to produce a left optical image of the abdominal cavity and a solid state image sensor such as a CCD image sensor 33L operative to convert the left optical image incident thereupon into video signals. The lens barrel 31L and the CCD image sensor 33L are assembled as one integral unit. Specifically, the lens barrel 31L is fixedly mounted to a lens holder 32L, and the CCD image sensor 33L is secured to the back of the lens holder 32L. Similarly, the right image pickup device 34R comprises an objective lens system 31LR built in a lens barrel 31R operative to produce a right optical image of the abdominal cavity and a solid state image sensor such as a CCD image sensor 33R operative to convert the right optical image incident thereupon into video signals. The lens barrel 31R and the CCD image sensor 33R are assembled as one integral unit such that the lens barrel 31R is fixedly mounted to a lens holder 32R and the CCD image sensor 33R is secured to the back of the lens holder 32R.

As is well known in the art, if the right and left image pickup devices 34R and 34L are fixedly installed side by side in the cylindrical sheath 35 with the respective CCD image sensors 33L and 33R put out of alignment in horizontal scanning direction, the properly-equipped viewer will perceive the image on a monitor connected to the system as a three-dimensional image with sensuously offensive stereopsis. For this reason, it is necessary to adjust the left image pickup devices 34L and 34R installed in the cylindrical sheath 35 so as to put the respective CCD image sensors 33L and 33R well aligned in horizontal scanning direction. Further, in the case where an electronic endoscope is provided with a single image pickup device like a laparoscope which produces a two-dimensional image for observational purposes only, it is desirable in light of even illumination or non-irregularity of illumination to set out a cylindrically tubular-shaped light guide surrounding the image pickup device that is positioned in alignment with a longitudinal center axis of the sheath of the endoscope such as shown in Figure 9. However, since it is essential for the electronic endoscope 30 used in the stereoscopic endoscope system to arrange the right and left image pickup devices 34R and 34L side by side in a diametrical direction or straight line passing through both optical axes of the right and left image pickup devices 34R and 34L, the electronic endoscope 30 has to have a cylindrical sheath 35 increased in its outer diameter if employing a cylindrically tubular-shaped light guide surrounding the right and left image pickup devices 34R and 34L similarly to the electronic endoscope that produces a two-dimensional image for observational purposes. This configuration comes into conflict with the demand that the cylindrical sheath 35 be as thin as possible.

The electronic endoscope 30 with a cylindrical sheath made as thin as possible is realized by employing a mounting structure for mounting the right and left image pickup devices 34R and 34L in the cylindrical sheath 35 at the distal end 11. The mounting structure includes a mounting fixture 36 basically comprising a solid cylindrical block having a relatively small thickness and approximately the same outer diameter as the inner diameter of the cylindrical sheath 35. The mounting fixture 36 has a pair of mounting bores 36H arranged side by side in a diametrical direction and is partially cut off in a plane parallel to an axial plane including center axes of the mounting bores 36H at each of opposite sides of the mounting bores 36H in a direction perpendicular to the axial plane. It is preferable to determine the outer diameter of the mounting fixture 36H such that the mounting fixture 36H is dovetailed or engaged with the cylindrical sheath 35. In an assembling process of the electronic endoscope 30, the right and left lens barrels 31R and 31L are fitted in and secured within the mounting bores 36H, respectively, of the mounting fixture 36 in advance and, then, the mounting fixture 36 is fitted into the cylindrical sheath 35 from the distal end 11.

When the mounting fixture 36 partially cut off in planes parallel to the axial plane at the opposite sides of the mounting bores 36H is tightly fitted in the cylindrical sheath 35, there are left arched spaces 35U and 35D, smaller in depth than a semicircular arch, between the cylindrical sheath 35 and the mounting fixture 36 on opposite sides of the mounting fixture 36. A light guide 37U in a form having a cross-section in conformity or similar to the arched space 35U extends through the cylindrical sheath 35 and is fitted in and secured within the arched space 35U at the distal end 11. Similarly, a light guide 37D a form having a cross-section in conformity or similar to the arched space 35D extends through the cylindrical sheath 35 and is fitted in and secured within the arched space 35D at the distal end 11. The light guides 37U and 37D are completely identical in structure and function. Each of the light guides 37U and 37D is made up of a great number of fibers bundled and bonded together with, for example, an epoxy resin adhesive so as to form an arched cross-section and completely identical in structure and function. In this embodiment, it is preferred to form a fiber bundle by shaping and firmly bonding fibers in an arched shape in an end region of the fiber bundle spatially overlapping the image pickup devices 34R and 34L and leaving them bundled but separate from one another in the remaining region.

As clearly seen in Figures 1 and 3, the light guides 37U and 37D at their distal ends are put substantially in plane with the distal end 11 of the cylindrical sheath 35.

The electronic endoscope 30 may or may not be provided with two light guides separately on opposite sides of the mounting fixture 36. In the case where the electronic endoscope 30 is provided with a single light guide, the mounting fixture 36 is partially cut off in a plane parallel to the axial plane at either one of the opposite sides of the mounting bores 36H so as to leave an arched space between the cylindrical sheath 35 and the mounting fixture 36 on the corresponding side of the mounting fixture 36.

Basically, as is known in the art, the stereoscopic endoscope system including the electronic endoscope having a pair of right and left image pickup devices can produce a three-dimensional image having stereoscopic depth when the right and left image pickup devices are positioned with their optical axes inclined with respect to each other so as to provide a predetermined angle of convergence. However, it is not always asseverated that such an inclined formation of the right and left image pickup devices is advantageous from the aspect of space availability and/or configuration.

In these circumstances, the electronic endoscope of the present invention is provided with an anterior lens element operative to provide an angle of convergence according to distance from the right and left objective lens systems 31LR and 31LL to an object to be viewed that is appropriate to provide the viewer with appropriate perceptible stereopsis on the image on the monitor. The anterior optical element comprises a converging lens 38 forming a part of both of the right and left objective lens systems 31LR and 31LL, which is preferably a convex lens and more preferably a plano-convex lens, secured to the mounting fixture 36. Specifically, as shown in Figure 1, the converging lens 38, that is partly cut off in substantially conformity with a front shape of the mounting fixture 36, is firmly fitted between and cemented, or otherwise secured, to the light guides 37U and 37D at the distal end 11 of the cylindrical sheath 35. In this instance, light blocking means 100U and 100D are applied at interfaces between the conversion lens 38 and the light guides 37U and 37D, respectively, to avoid a causes of an occurrence of flair due to stray light from the light guides 37U and 37D entering the objective lens systems 31LR and 31LL through the periphery of the conversion lens 38. The application of light blocking means 100U and 100D has a significant effect on improving image quality. The light blocking means 100U and 100D may comprise an adhesive containing a light shielding material such as a black pigment that is used to cement the converging lens 38 and the light guides 37U and 37D together or a light shielding film. The light shielding material or the light shielding film are known in various forms and may take any form well known in the art.

According to the configuration of the electronic endoscope 30 described above, it is allowed to employ a cylindrical sheath 35 significantly reduced in diameter although the cylindrical sheath 35 has a pair of the right and left image pickup devices 34R and 34L each comprising the objective lens system 31LR or 31LL built in the lens barrel 31R or 31L and the CCD image sensor 33R or 33L incorporated therein. For example, when comparing with the conventional electronic endoscope with a pair of image pickup devices which has a double-barreled sheath having inner and outer diameters D1 and D2 as shown in Figure 9 and 10, the cylindrical sheath 35 of the electronic endoscope 30 can be reduced in outer diameter to the inner diameter D1.

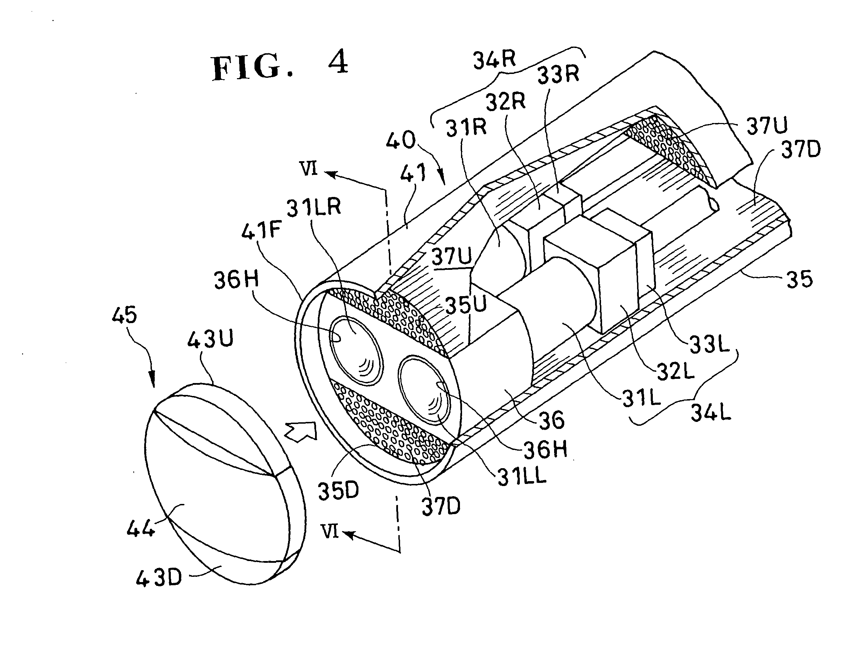

Figures 4 to 6 show an electronic endoscope 40 for stereoscope systems according to alternative embodiment of the present invention in which transparent protective plates for protecting the light guides 37U and 37D are incorporated with an intention to avoid drawbacks inherent to the light guides 37U and 37D resulting from that it is often the case of using resin adhesives such as epoxy resin adhesives to bond a great number of fibers together and, in this instance, the adhesive has a comparatively high proportion of area in a cross section of the fiber bundle. That is, when the electronic endoscope 40 with the light guides 37U and 37D whose distal end surfaces are barely formed or exposed as shown in, for example, Figure 1 is used as a laparoscope, at least the elongated cylindrical sheath is always cleaned and disinfected after use of the electronic endoscope. Consequently, the adhesive in the light guide 37U and 37D deteriorates on contact with chemical solutions such as cleaning and disinfectant solutions. Such adhesive deterioration leads to optical and/or mechanical local damages and/or deterioration of air-tightness of the distal ends of the light guide 37U and 37D which cause failure of image quality and a problem of reliability of the electronic endoscope depending on how the adhesive deterioration progresses.

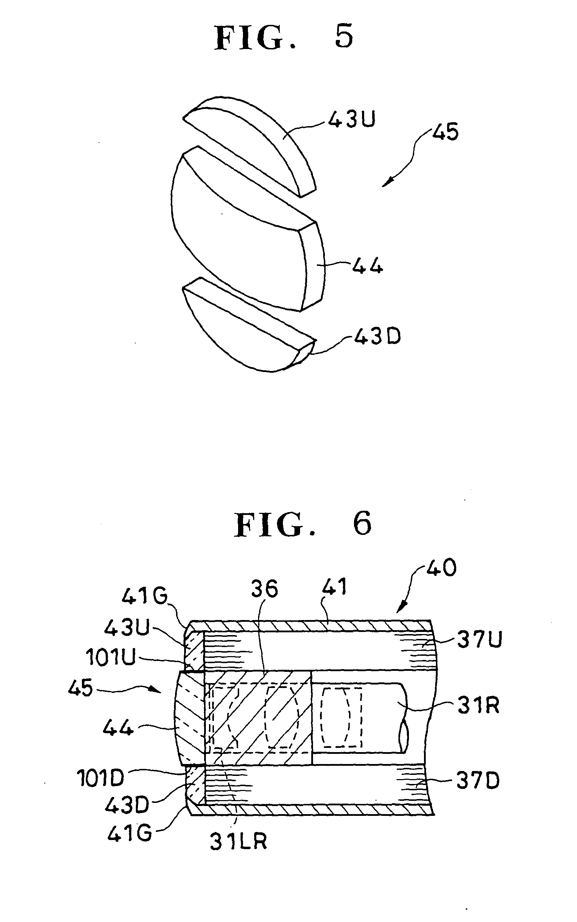

Specifically, the electronic endoscope according to the alternative embodiment 40 includes light guides 37U and 37D and a mounting fixture 36 with right and left image pickup devices 34R and 34L fitted in and secured within mounting bores 36H thereof, respectively, similar to those of the electronic endoscope 40 of the previous embodiment shown in Figures 1 to 3. The right image pickup device 34R comprises an objective lens system 31LR built in a lens barrel 31R and a CCD image sensor 33R optically connected to each other by means of a lens holder 32R. Similarly, the left image pickup device 34L comprises an objective lens system 31LL built in a lens barrel 31L and a CCD image sensor 33L optically connected to each other by means of a lens holder 32L. The light guides 37U and 37D are fitted in and secured within arched spaces 35U and 35D, respectively, formed between the cylindrical sheath 31 and the mounting fixture 36. However, in this instance, the light guides 37U and 37D at their distal end and the mounting fixture 36 at its front end are located in plane with each other and kept away from but in close proximity to the distal end of the elongated cylindrical sheath 31 so as thereby to leave an anterior annular space in the cylindrical sheath 31 before the mounting fixture 36 and the light guides 37U and 37D. The electronic endoscope 40 further includes a circular anterior optical element 45 fitted in the annular space of the cylindrical sheath 31 as shown in Figure 6. The circular anterior optical element 45 comprises a converging lens 44 in conformity with the mounting mixture 36 and transparent protective parallel plates 43U and 43D in conformity with the arched spaces 35U and 35D, respectively, as clearly shown in Figure 5. These converging lens 44 and arched transparent protective parallel plates 43U and 43D are complementary to form a substantially complete circular element and cemented to one another with an adhesive so as thereby to form an integral anterior optical element 45. The circular anterior optical element 45 is fitted in the annular space of the cylindrical sheath 31 from the distal end and secured to the cylindrical sheath 31 with an adhesive. It is preferred to mechanically bear down the anterior optical element 45 with a peripheral edge 41G of the cylindrical sheath 31 clinched at least partly, and desirably entirely, along the periphery of the distal end radially inwardly as shown in Figure 6. In this instance, it is desirable for the anterior optical element 45 to have a chamfer formed along its frontal periphery. Otherwise, the elongated cylindrical sheath 31 may be provided with an annular flange extending radially inwardly from the distal end thereof. In this instance, the mounting fixture with the right and left image pickup means attached thereto is installed into the elongated cylindrical sheath only from the proximal end.

In this instance, light blocking means 101U and 101D are applied at interfaces between the conversion lens 44 and the transparent protective parallel plates 43U and 43D, respectively, to prevent light emanating from the light guides 37U and 37D from directly entering the converging lens 44 through its periphery. The light blocking means 101U and 101D may comprise a light shielding material such as a black pigment contained in the adhesive that is used to cement the converging lens 44 and the transparent protective parallel plates 43U and 43D together or a light shielding film. The light shielding material or the light shielding film are known in various forms and may take any form well known in the art.

While the configuration of the electronic endoscope 40 according to the alternative embodiment is of great advantage in employing the cylindrical sheath 31 significantly reduced in outer diameter resulting from high space availability for the light guide 37U and 37D. Besides this advantage, the electronic endoscope 40 avoids possible deterioration of the adhesive in the light guide 37U and 37D at their distal end due to direct contact with chemical solutions such as cleaning and disinfectant solutions. This is of great advantage in enhancing the durability of the light guide 37U and 37D, and hence the electronic endoscope 40. Furthermore, although axes of the image pickup devices come close to each other due to a reduced diameter of the cylindrical sheath 31, nevertheless, illumination light and image bearing light are finely isolated from each other by the light shielding means. Consequently, the electronic endoscope 40 produces high quality images.

Furthermore, the configuration that the anterior optical element is mechanically borne down by clinching partially a peripheral edge of the sheath at distal end radially inwardly prevents the adhesive with which the anterior optical element is cemented to the sheath from brought into contact with chemical solutions during cleaning and/or disinfection, thereby protecting the distal ends of the light guides against damages, and besides, provides significantly reliable and enhanced supporting strength.

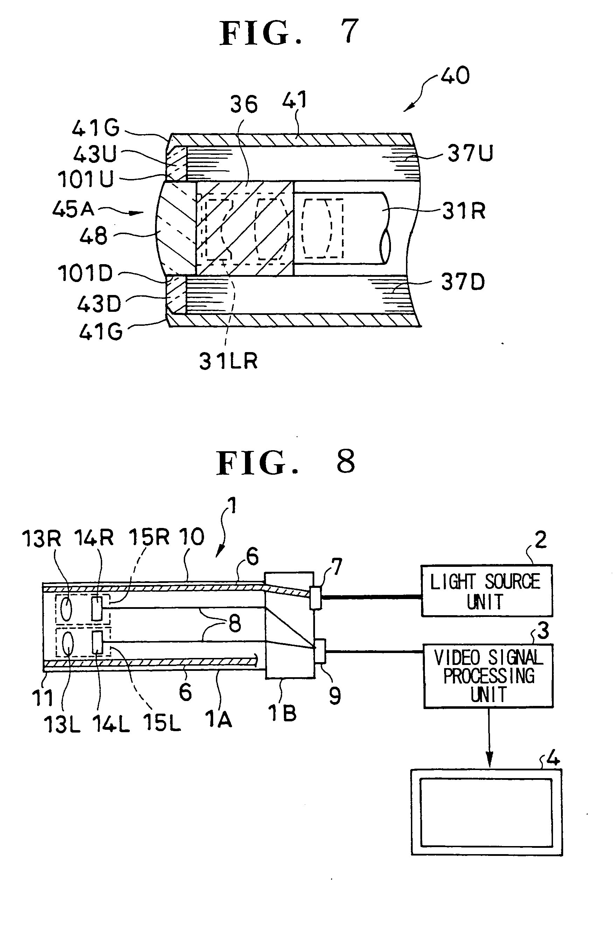

Figure 7 shows a variant of the electronic endoscope 40 of the alternative embodiment which has a modified distal end configuration. As shown, light guides 37U and 37D are located in plane with each other and separately from but in close proximity to the distal end of the cylindrical sheath 31 so as thereby to leave an anterior annular space in the cylindrical sheath 31 for flat transparent protective parallel plates 43U and 43D in the cylindrical sheath 31. Besides the light guides 37U and 37D, a mounting fixture 36 is set back toward the proximal end from but in close proximity to the distal ends of the light guides 37U and 37D. The variant configuration enables the use of a converging lens 48 having an increased axial thickness and allows an anterior optical element 45A to be fixedly supported between the light guides 37U and 37D additionally by the cylindrical sheath 31. Consequently, positioning and fixing the anterior optical element 45A is more easily be performed.

In the embodiments described above, though the converging lenses 38, 44 and 48 as the anterior optical element or forming part of the anterior optical element have been exemplified in the form of a plano-convex lens, it may be of a meniscus type having a large curvature radius at an exit side. The transparent protective plates 43U and 43D which have been exemplified in the form of a parallel plate may be replaced with a simple projection lens. Further, the electronic endoscope 30, 40 may incorporate at least one light guide disposed in an arched space defined between the mounting fixture and the sheath on either one side of the mounting fixture in a direction perpendicular to the axial plane including center axes of the mounting bores of the mounting fixture.

## Claims

1. An electronic endoscope (30; 40) for stereoscopic endoscope systems that includes an elongated cylindrical sheath (35) for insertion into an interior of a hollow body in which illumination guide means (37U, 37D) optically connected to a light source for illuminating said interior of said hollow body and a pair of right and left image pickup means (34R, 34L) each comprising an objective lens system (31LR, 31LL) and a solid state image sensor (33R, 33L) for gathering right and left optical images of said hollow body, respectively, and converting the right and left optical images into video signals to display said images of said hollow body on a monitor perceivable as a three-dimensional image with stereoscopic effect a properly-equipped viewer of the system, **characterized by**:
a mounting fixture (36) that has a pair of mounting bores (36H) arranged side by side to hold said right and left solid state image pickup means (34R, 34L) therein, respectively, so as to locate said right and left solid state image pickup means (34R, 34L) side by side in a diametrical direction of said elongated cylindrical sheath (35) and in proximity to an distal end of said elongated cylindrical sheath (35) when it is fitted in said elongated cylindrical sheath (35) and is shaped so as to form an arched space (35U, 35D) between said mounting fixture (36) and said elongated cylindrical sheath (35) at least one side thereof in a direction perpendicular to said diametrical direction for receiving said illumination guide means (37U, 37D) therein when it is fitted in said elongated cylindrical sheath (35).

2. An electronic endoscope (30; 40) for stereoscopic endoscope systems as defined in claim 1, **characterized by** a single converging lens (38; 44; 48) through which said right and left image pickup means (34R, 34L) gather said right and left optical images of said hollow body, wherein said single converging lens (38; 44; 48) is configured in conformity with an anterior extremity of said mounting fixture (36) and fixedly installed in said elongated cylindrical sheath (35) at said distal end.

3. An electronic endoscope (40) for stereoscopic endoscope systems as defined in claim 1 or 2, **characterized by** a transparent protective plate (43U, 43D) for protecting a distal end of said illumination guide means (37U, 37D), wherein said transparent protective plate (43U, 43D) is shaped in conformity with said arched space (35U, 35D) and installed in said elongated cylindrical sheath (35) at said distal end.

4. An electronic endoscope (40) for stereoscopic endoscope systems as defined in claim 2 or 3, wherein said single converging lens (44; 48) and said transparent protective plate (43U, 43D) are complementary to each other to form a substantially completely circular anterior optical element (45; 45A) in conformity with a cross section of said elongated cylindrical sheath (35) at said distal end.

5. An electronic endoscope (40) for stereoscopic endoscope systems as defined in any one of the preceding claims 2 through 4, **characterized in that** said converging lens (44; 48) and said transparent protective plate (43U, 43D) are cemented to each other to form said substantially circular optical element (45; 45A).

6. An electronic endoscope (30) for stereoscopic endoscope systems as defined in any one of the preceding claims 2 through 5, **characterized in that** said circular optical element (45; 45A) is fixedly borne down by a peripheral edge (41G) of said elongated cylindrical sheath (35) clinched radially inwardly at said distal end (11).

7. An electronic endoscope (30; 40) for stereoscopic endoscope systems as defined in any one of the preceding claims 2 through 6, **characterized by** light blocking means (100U, 100D; 101U, 101D) disposed at an interface between said converging lens (38) and said illumination guide means (37U, 37D) or between said converging lens (44; 48) and said transparent protective plate (43U, 43D) for preventing illumination light emanating from said illumination light guide (37U, 37D) from entering said converging lens (38; 44; 48) .

8. An electronic endoscope (30) for stereoscopic endoscope systems as defined in claim 7, **characterized in that** said light blocking means (100U, 100D; 101U, 101D) comprises either one of a light blocking film disposed at said interface, a black coating applied to said conversing lens (38; 44; 48), and a black adhesive for cementing said converging lens (38; 44; 48) and said illumination guide means (37U, 37D) or said transparent protective plate (43U, 43D) together.
